(19) Europäisches Patentamt
European Patent Office
Office européen des brevets

(11) Veröffentlichungsnummer : **0 053 326**
**B1**

(12) # EUROPÄISCHE PATENTSCHRIFT

(45) Veröffentlichungstag der Patentschrift : **08.02.84**

(51) Int. Cl.$^3$ : **C 07 C121/34**, C 07 C120/00

(21) Anmeldenummer : **81109756.7**

(22) Anmeldetag : **19.11.81**

(54) **Verfahren zur Herstellung von Pivaloylcyanid.**

(30) Priorität : **01.12.80 DE 3045181**

(43) Veröffentlichungstag der Anmeldung : **09.06.82 Patentblatt 82/23**

(45) Bekanntmachung des Hinweises auf die Patenterteilung : **08.02.84 Patentblatt 84/06**

(84) Benannte Vertragsstaaten : **AT BE CH DE FR GB IT LI NL**

(56) Entgegenhaltungen :
**DE-A- 2 708 182**
**DE-A- 2 708 183**
**DE-B- 2 660 344**

(73) Patentinhaber : **BAYER AG**
**Zentralbereich Patente, Marken und Lizenzen**
**D-5090 Leverkusen 1, Bayerwerk (DE)**

(72) Erfinder : **Findeisen, Kurt, Dr.**
**In der Follmühle 10**
**D-5068 Odenthal 2 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 053 326 B1

Jouve, 18, rue St-Denis, 75001 Paris, France

# Verfahren zur Herstellung von Pivaloylcyanid

Die Erfindung betrifft ein Verfahren zur kontinuierlichen Herstellung von Pivaloylcyanid durch Umsetzung von Pivalinsäureanhydrid mit wasserfreier Blausäure unter bestimmten Verfahrensbedingungen. Pivaloylcyanid kann als Zwischenprodukt zur Herstellung von bekannten herbiziden Wirkstoffen verwendet werden.

Es ist bereits bekannt geworden, daß man Pivaloylcyanid dadurch erhalten kann, daß man Pivalinsäureanhydrid mit wasserfreier Blausäure mehrere Stunden in einem Autoklaven auf 250 °C erhitzt und das erhaltene Reaktionsgemisch fraktioniert destilliert ; die Ausbeute beträgt bei einem 5 Mol-Ansatz 88 % der Theorie (vgl. DE-PS 26 14 241, Beispiel 4). Jedoch bedarf es zur Erzielung dieses Ergebnisses der Durchführung von Einzelansätzen im Autoklaven, und eine kontinuierliche Ausgestaltung dieses Verfahrens wäre mit sehr erheblichem technischem Aufwand verbunden. Außerdem hat sich gezeigt, daß bei größeren Ansätzen eine Verminderung der Ausbeute eintritt.

Es wurde nun gefunden, daß man Pivaloylcyanid der Formel

$$(CH_3)_3C—CO—CN \qquad (I)$$

in kontinuierlicher Verfahrensweise durch Umsetzung von Pivalinsäureanhydrid, $(CH_3)_3C—CO—O—CO—C(CH_3)_3$, mit wasserfreier Blausäure in sehr hoher Ausbeute und Reinheit herstellen kann, indem man die Umsetzung in Gegenwart eines komplexen Alkali- oder Erdalkali-Kupfer-Cyanids als Katalysator und in Gegenwart eines inerten, aprotischen organischen, oberhalb von 210 °C siedenden Lösungsmittels als Verdünnungsmittel bei Temperaturen zwischen 180 und 240 °C unter Normaldruck durchführt, dergestalt, daß man zu einer bei der Reaktionstemperatur gerührten Suspension des Katalysators in dem Verdünnungsmittel gleichzeitig und kontinuierlich das Pivalinsäureanhydrid zutropfen läßt und die Blausäure gasförmig einführt und daß man das rohe, im wesentlichen aus Pivaloylcyanid, Pivalinsäure und nicht-umgesetzter Blausäure bestehende Produktgemisch kontinuierlich aus dem Reaktionsgefäß abdestilliert, die nicht-umgesetzte Blausäure durch Verdampfung abtrennt und im Kreislauf in das Reaktionsgefäß zurückführt und das Pivaloylcyanid aus dem anfallenden rohen Produktgemisch durch fraktionierte Vakuumdestillation abtrennt.

Es war ferner bekannt, daß die Synthesen von Acylcyaniden aus Carbonsäureanhydriden durch Umsetzung mit Blausäure oder Alkalicyaniden nicht oder nur sehr schlecht gelingen, wenn man im alkalischen Medium oder in Gegenwart alkalischer Katalysatoren arbeitet, da in alkalischem Milieu dimere Acylcyanide gebildet werden (vgl. Angewandte Chemie *68*, S. 425-435 (1956), insbesondere S. 426 und 434). Im Hinblick auf diesen Stand der Technik ist es als überraschend zu bezeichnen, daß die Umsetzung von Pivalinsäureanhydrid mit Blausäure durch die genannten, basischen Komplexsalze katalysiert werden kann, so daß im Vergleich zu dem aus DE-PS 26 14 241 (Beispiel 4) vorbekannten Verfahren (3 Stunden bei 250 °C und entsprechendem Überdruck im Autoklaven) bei niedrigeren Temperaturen, wesentlich kürzeren Verweilzeiten (in der Größenordnung von wenigen Minuten) und bei Normaldruck gearbeitet werden kann, wobei gleichzeitig höhere Ausbeuten erzielt werden und ein großer Durchsatz möglich ist. Dabei ist es einerseits überraschend, daß die aufgefundene katalytische Wirksamkeit der genannten Komplexsalze im Prinzip unbegrenzt erhalten bleißt, daß also die Salze nicht mit Pivalinsäureanhydrid zu Pivaloaten + Pivaloylcyanid abreagieren und dadurch desaktiviert werden ; andererseits überrascht es, daß trotz Anwesenheit basischer Agentien kein dimeres Pivaloylcyanid gebildet wird, und daß die Blausäure nicht polymerisiert. — Besonders überraschend ist außerdem der Befund, daß andere niederaliphatische Carbonsäureanhydride in entsprechender Weise nur mit wesentlich geringeren Ausbeuten umgesetzt werden können.

Der Reaktionsverlauf kann durch das folgende Formelschema wiedergegeben werden :

$$(CH_3)_3C—CO—O—CO—C(CH_3)_3 + HCN \xrightarrow[\text{Verdünnungsmittel}]{\text{180-240 °C Katalysator}}$$

$$(CH_3)_3C—OOOH + (CH_3)_3C—CO—CN$$

Es hat sich gezeigt, daß anstelle von Pivalinsäureanhydrid auch die äquivalente Menge Pivaloylchlorid eingesetzt werden kann. In diesem Falle wird anstelle von Pivalinsäure Chlorwasserstoff freigesetzt, der in geeigneter Weise abgeführt werden muß.

Die erfindungsgemäße Umsetzung wird in Gegenwart eines komplexen Alkali- oder Erdalkali-Kupfer-Cyanids als Katalysator durchgeführt. Solche Komplexverbindungen entstehen durch Umsetzung von Alkali- und Erdalkali-Cyaniden mit Kupfer-(I)-cyanid. Als Beispiel wird die Bildung von Natrium-Kupfer-Cyanid (Natrium-tetracyanocuprat (I)) angegeben :

$$3NaCN + CuCN \longrightarrow Na_3[Cu(CN)_4]$$

Weitere Beispiele für geeignete Komplexverbindungen dieser Art sind $K_3[Cu(CN)_4]$, $Ca_3[Cu(CN)_4]_2$ und $Ba_3[Cu(CN)_4]_2$.

Besonders zweckmäßig ist es, die als Katalysator einzusetzende Komplexverbindung vor Beginn der kontinuierlichen Umsetzung im Reaktor aus den Einzelkomponenten frisch herzustellen.

Die genannten Katalysatoren sind von langer Lebensdauer und regenerieren sich während der Reaktion von selbst. Ein Nachlassen der Aktivität dieser Katalysatoren ist bisher in keinem Fall beobachtet worden.

Die erfindungsgemäße Umsetzung wird außerdem in Gegenwart eines inerten, aprotischen organischen, oberhalb von 210 °C siedenden Lösungsmittels als Verdünnungsmittel durchgeführt. Als derartige Verdünnungsmittel seien beispielhaft genannt: Diphenylether, 1,3,5-Triisopropylbenzol, 1,2,4-Trichlorbenzol, Benzoesäurepropylester, 2,3,4-Trichlortoluol.

Bevorzugt wird Diphenylether verwendet.

Die Reaktionstemperatur kann in einem größeren Bereich variiert werden. Im allgemeinen arbeitet man, wie oben angegeben, bei Temperaturen zwischen 180 und 240 °C, vorzugsweise zwischen 195 und 225 °C.

Es hat sich als zweckmäßig erwiesen, bei der Durchführung des Verfahrens pro Liter Verdünnungsmittel etwa 0,1 bis 0,2 Mol des Katalysators einzusetzen. Unter diesen Bedingungen können etwa 1 bis 2 Mol Pivalinsäureanhydrid pro Stunde und überschüssige Blausäure (z. B. 5 bis 8 Mol pro Stunde) zugeführt werden, wobei das Pivalinsäureanhydrid etwa zu 90 % umgesetzt wird. Die überschüssige, nicht-umgesetzte Blausäure wird im Kreislauf in das Vorratsgefäß und schließlich in den Reaktor zurückgeführt.

Das Produktgemisch, das aus äquivalenten Mengen an Pivaloylcyanid (I) und Pivalinsäure besteht, wird kontinuierlich aus dem Reaktor abdestilliert; hierbei gehen die nicht-umgesetzte Blausäure sowie geringe Mengen an nicht-umgesetztem Pivalinsäureanhydrid ($< 10$ %) und gegebenenfalls an Verdünnungsmittel mit über.

Zunächst wird die Blausäure durch Verdampfen abgetrennt und, wie bereits beschrieben, im Kreislauf zurückgeführt. Das Pivaloylcyanid (I) und die übrigen Bestandteile des Rohdestillats werden anschließend durch fraktionierte Destillation aufgetrennt und isoliert und gegebenenfalls weiterverarbeitet.

Das nach dem erfindungsgemäßen Verfahren herstellbare Pivaloylcyanid der Formel (I) ist ein wertvoller Ausgangsstoff z. B. zur Synthese von 1,2,4-Triazin-5-onen, welche hervorragende herbizide Eigenschaften besitzen (vgl. z. B. DE-PS 17 95 784). So läßt sich Pivaloylcyanid beispielsweise in den herbiziden Wirkstoff 3-Methylthio-4-amino-6-tert.-butyl-1,2,4-triazin-5-on überführen, durch folgende Reaktionsfolge:

Saure Hydrolyse (vgl. Angew. Chem. *68*, S. 430 (1956)), Umsetzung der gebildeten α-Ketosäure — 3,3-Dimethyl-2-oxo-buttersäure — mit Thiocarbohydrazid (vgl. Chem. Ber. *97*, Seiten 2173-8 (1964)) und Methylierung des gebildeten 3-Mercapto-4-amino-6-tert.-butyl-1,2,4-triazin-5-ons, z. B. mittels Methylbromid (vgl. DE-OS 27 29 761); vgl. ferner DE-OS 30 03 541.

Das erfindungsgemäße Verfahren soll durch das nachfolgende Beispiel veranschaulicht werden:

## Beispiel 1

### Versuchsanordnung

Die Durchführung geschieht in einem mit Öl beheizten 2-l-Vierhalskolben. Der Kolben besitzt folgende Aufsätze: Rührer, Einleitungskapillare (für Blausäure; über einen Vorverdampfer und eine Dosierpumpe mit einem Blausäure-Vorratsgefäß verbunden), Tropftricher (für Pivalinsäureanhydrid), Thermometer und eine mit Raschigringen gefüllte Silbermantelkolonne mit Kolonnenkopf. An den Kolonnenkopf schließt sich ein Kühler an, der in eine weitere, gleich große Vierhalskolbenrührapparatur, versehen mit Bodenablaß, mündet. Dieser zweite Kolben wird auf 90 bis 95 °C gehalten. Auf einem Seitenhals des zweiten Kolbens steht eine mit warmen Wasser (30 °C) beschickte Kolonne, auf der sich eine mit Kühlsole gekühlte Destillationsbrücke befindet. In der Brücke wird die nicht umgesetzte Blausäure kondensiert und in das Vorratsgefäß zurückgeführt. Mit einer gekülten Dosierpumpe wird die Blausäure über einen Vorverdampfer und durch die oben erwähnte Kapillare in das Reaktionsgefäß gepumpt.

### Versuchsdurchführung

In den Reaktor (2 l-Vierhalskolben) werden 600 ml Diphenylether und — zur Erzeugung des Katalysators, $Na_3[Cu(CN)_4]$ — 8,9 g (0,1 Mol) Kupfer-I-cyanid und 15 g (0,3 Mol) 98 %iges Natriumcyanid vorgelegt. Bei der Reaktionstemperatur von 205 bis 215 °C wird über die Dosierpumpe, Vorverdampfer und Einleitungskapillare Blausäuredampf eingegeben (etwa 250 bis 300 ml (6,5 bis 7,5 Mol) HCN pro Stunde). Aus dem Vorratstropfrichter für Pivalinsäure-anhydrid tropfen ca. 250 bis 280 g (1,35 bis 1,5 Mol) pro Stunde zu. Die aus dem Reaktionsgefäß austretenden Produkte und die überschüssige Blausäure werden im Kolonnenkopf auf eine Temperatur von 110 bis 115 °C eingestellt. In dem sich

anschließenden Vierhalskolben mit Bodenablaß wird die überschüssige Blausäure ausgetrieben und im Vorratsgefäß aufgefangen.

Das Reaktionsprodukt wird über den Bodenablaß entfernt und zur Destillation gebracht.

Innerhalb von 10 Stunden werden 2 536 g (13,63 Mol) Pivalinsäureanhydrid zugetropft.

Aufgefangene Menge Rohdestillat (nach 10 Stunden) : 3 082 g.

Zur Trennung der Reaktionsprodukte wird das Rohdestillat über eine Füllkörper-Silbermantelkolonne mit aufgesetztem Kolonnenkopf destilliert.

Ausbeute : 1 442 g Pivaloylcyanid (= 95,3 % der Theorie), Siedepunkt : 53-55 °C/130-140 mbar.

Außerdem wurden erhalten :

1 316 g Pivalinsäure (= 94,7 % der Theorie), Siedepunkt : 163 °C bzw. 70 °C/18,6 mbar ;

154 g Pivalinsäureanhydrid (ca. 6 %, nicht umgesetzt), Siedepunkt : 190 °C ;

120 g Diphenylether, Siedepunkt : 252 °C bzw. 127 °C/13,3 mbar.

**Ansprüche**

1. Verfahren zur kontinuierlichen Herstellung von Pivaloylcyanid der Formel

$$(CH_3)_3C—CO—CN \quad (I)$$

durch Umsetzung von Pivalinsäureanhydrid mit wasserfreier Blausäure, dadurch gekennzeichnet, daß man die Umsetzung in Gegenwart eines komplexen Alkali- oder Erdalkali-Kupfer-Cyanids als Katalysator und in Gegenwart eines inerten, aprotischen organischen, oberhalb von 210 °C siedenden Lösungsmittels als Verdünnungsmittel bei Temperaturen zwischen 180 und 240 °C unter Normaldruck durchführt, dergestalt, daß man zu einer bei der Reaktionstemperatur gerührten Suspension des Katalysators in dem Verdünnungsmittel gleichzeitig und kontinuierlich das Pivalinsäureanhydrid zutropfen läßt und die Blausäure gasförmig einführt und daß man das rohe, im wesentlichen aus Pivaloylcyanid, Pivalinsäure und nicht-umgesetzter Blausäure bestehende Produktgemisch kontinuierlich aus dem Reaktionsgefäß abdestilliert, die nicht-umgesetzte Blausäure durch Verdampfung abtrennt und im Kreislauf in das Reaktionsgefäß zurückführt und das Pivaloylcyanid aus dem anfallenden rohen Produktgemisch durch fraktionierte Vakuumdestillation abtrennt.

2. Verfahren nach Anspruch 1, dadurch gekennzeichnet, daß man bei Temperaturen zwischen 195 und 225 °C arbeitet.

3. Verfahren nach Anspruch 1, dadurch gekennzeichnet, daß man als Katalysator das komplexe Cyanid der Formel $Na_3[Cu(CN)_4]$ oder $K_3[Cu(CN)_4]$ einsetzt.

4. Verfahren nach Anspruch 1, dadurch gekennzeichnet, daß man als Verdünnungsmittel Diphenylether einsetzt.

**Claims**

1. Process for the continuous preparation of pivaloyl cyanide of the formula

$$(CH_3)_3C—CO—CN \quad (I)$$

by reaction of pivalic acid anhydride with anhydrous hydrocyanic acid, characterised in that the reaction is carried out in the presence of an alkali metal complex copper cyanide or an alkaline earth metal complex copper cyanide as catalyst and in the presence of an inert, aprotic organic solvent, which boils above 210 °C, as diluent, at temperatures between 180 and 240 °C, and under normal pressure, in such a manner that, simultaneously and continuously, the pivalic acid anhydride is allowed to drop into, and the hydrocyanic acid is introduced in gaseous form into, a suspension of the catalyst in the diluent, which is stirred at the reaction temperature, and that the crude product mixture, consisting essentially of pivaloyl cyanide, pivalic acid and unreacted hydrocyanic acid, is continuously distilled off from the reaction vessel, the unreacted hydrocyanic acid is separated off by evaporation and is recycled into the reaction vessel, and the pivaloyl cyanide is separated from the precipitated crude product mixture by fractional distillation in vacuo.

2. Process according to Claim 1, characterised in that the reaction is carried out at temperatures between 195 and 225 °C.

3. Process according to Claim 1, characterised in that the complex cyanide of the formula $Na_3[Cu(CN)_4]$ or $K_3[Cu(CN)_4]$ is employed as the catalyst.

4. Process according to Claim 1, characterised in that diphenyl ether is employed as the diluent.

**Revendications**

1. Procédé de production continue de cyanure de pivaloyle de formule

$$(CH_3)_3C—CO—CN \quad (I)$$

par réaction d'anhydride d'acide pivalique avec l'acide cyanhydrique anhydre, caractérisé en ce qu'on conduit la réaction en présence d'un cyanure complexe de métal alcalin ou alcalino-terreux et de cuivre comme catalyseur et en présence d'un solvant organique aprotique inerte bouillant au-dessus de 210 °C, utilisé comme diluant, à des températures comprises entre 180 et 240 °C à la pression normale, en ajoutant en même temps et continuellement goutte à goutte l'anhydride d'acide pivalique à une suspension du catalyseur dans le diluant, agitée à la température de réaction, et en introduisant l'acide cyanhydrique à l'état gazeux, et on chasse continuellement par distillation du mélange réactionnel le mélange brut de produits essentiellement formé de cyanure de pivaloyle, d'acide pivalique et d'acide cyanhydrique n'ayant pas réagi, on sépare l'acide cyanhydrique n'ayant pas réagi par évaporation et on le recycle dans le récipient de réaction, et on sépare le cyanure de pivaloyle du mélange brut de produits obtenus, par distillation fractionnée sous vide.

2. Procédé suivant la revendication 1, caractérisé en ce qu'on opère à des températures comprises entre 195 et 225 °C.

3. Procédé suivant la revendication 1, caractérisé en ce qu'on utilise comme catalyseur le cyanure complexe de formule $Na_3[Cu(CN)_4]$ ou $K_3[Cu(CN)_4]$.

4. Procédé suivant la revendication 1, caractérisé en ce qu'on utilise l'éther de diphényle comme diluant.